# EUROPEAN PATENT SPECIFICATION

(11) **EP 0 508 310 B1**
(45) Date of publication and mention of the grant of the patent: **13.03.1996**
(21) Application number: 92105712.1
(22) Date of filing: 02.04.1992
(51) Int. Cl.: A61K 31/40, A61K 9/02, A61K 47/10, A61K 47/32

(54) **Pharmaceutical composition for rectal administration, which contains a 2-oxindole-1-carboxamide derivative**
2-Oxindol-1-carboxamidderivat enthaltendes Arzneimittel zur rektalen Verabreichung
Composition pharmaceutique pour l'administration rectale contenant un dérivé du 2-oxindole-1-carboxamide

(30) Priority: 08.04.1991 DE 4111305
(43) Date of publication of application: 14.10.1992
(73) Proprietor: Heinrich Mack Nachf., D-89252 Illertissen (DE)
(72) Inventor: Fries, Walter, W-7918 Illertissen (DE); Pfaff, Günther, Dr., W-7918 Illertissen (DE); Pfitzner, Carl Jörg, W-7918 Illertissen (DE); Simon, Gerhard, Dr., W-7918 Illertissen (DE)
(74) Representative: Lederer, Franz, Dr.

(56) References cited:
- EP-A- 0 156 603
- EP-A- 0 277 738
- EP-A- 0 337 627

## Description

Pharmaceutical agents are administered in various forms such as tablets, solutions for injection or suppositories. Suppositories are pharmaceutical compositions which are introduced into the rectum. They usually consist of a suppository base, the agent or agents and, if necessary, additional inactive ingredients. The agent is released after the suppository has melted or dissolved. This entails the necessity for the agent contained in the suppository first to be dissolved in the rectal fluid, which is normally extremely little (only 1-3 ml), in order to be able to be absorbed through the rectal wall.

2-Oxindole-1-carboxamide derivatives are described in US Patent 4556672, to which express reference is hereby made. These agents have, in particular, antiinflammatory and analgesic effects.

It has been found that 2-oxindole-1-carboxamide derivatives result, after rectal administration, in widely varying curves of blood levels. Differences arise owing to the fact that some patients are able to absorb the agent well, while others are able to absorb it only poorly. However, different curves of blood levels may result in unwanted side effects.

It is therefore desirable to make available a suppository which, on rectal administration, brings about approximately the same bioavailability of the agent as can be obtained after oral administration.

It is possible in this way to avoid the necessity for the individual suppository to contain a higher dose of the agent than the oral administration form, which might result in side effects on complete absorption by efficiently absorbing patients. Thus, a suppository which brings about complete absorption of the agent makes more accurate and reliable dosage of the agent possible.

It is possible to employ absorption promoters in order to achieve approximately the same bioavailability after rectal administration by comparison with oral administration; the compounds, which are also called absorption enhancers, make it possible for the agents to pass more quickly through the intestinal wall into the blood.

A list of absorption promoters which can be used is to be found in the citation "Enhancement of Rectal Drug Absorption" by E.J. van Hoogdalem, 1989. Various representatives of the absorption promoters mentioned in this citation were tested for efficacy for 2-oxindole-1-carboxamide derivatives. It was found in these tests that no more than a negligible improvement in the bioavailability could be achieved by the absorption promoters mentioned in the citation.

Even when absorption promoters known per se were employed it was necessary for the amount of agent incorporated into a suppository to be about 1.5 to 3 times an oral dose of agent in order to achieve equally high blood levels after rectal administration as after an oral dose. Thus, it was not possible, even on use of absorption promoters known per se, to abandon the requirement, which is known from the state of the art, of incorporating into a suppository the amount of agent which is 1.5 to 3 times an oral dose (H. Sucker, P. Fuchs and P. Speiser, Pharmazeutische Technologie [Pharmaceutical Technology], Thieme-Verlag, Stuttgart 1978, page 503).

It has now been found, surprisingly, that a considerable improvement in the bioavailability can be achieved by incorporating polyethylene glycol or polyvinylpyrrolidone into the suppositories, although neither Pluronics® nor polyols caused more than a negligible improvement in the bioavailability, and although other molecular-disperse solubilisers likewise caused an inconsiderable improvement in absorption.

Hence the present invention relates to pharmaceutical compositions for rectal administration, which contain a 2-oxindole-1-carboxamide derivative and, as absorption promoter, polyethylene glycol or polyvinylpyrrolidone.

The 2-oxindole-1-carboxamide derivatives which are preferably employed within the scope of the present invention are the compounds of the formula I 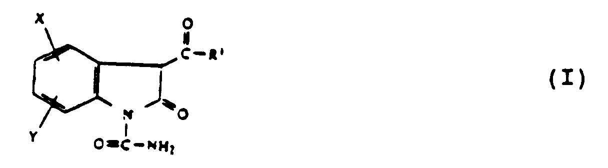
or pharmaceutically acceptable salts thereof, in which Y is hydrogen or chlorine, X is 5-chloro, 6-chloro, 5-fluoro, 6-fluoro, 5-trifluoromethyl or 6-trifluoromethyl, and in which R¹ is benzyl, 2-furyl, 2-thienyl, (2-furyl)methyl or (2-thienyl)methyl.

Compounds which are particularly preferably employed are those of the formula II 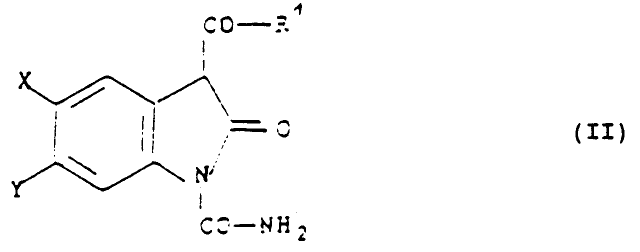
in which the substituents X, Y and R¹ have the following meanings, either
a) X = Cl, Y = H, R¹ = 2-thienyl or
b) X = F, Y = Cl, R¹ = 2-thienyl or
c) X = H, Y = Cl, R¹ = benzyl.

Very particularly preferably employed within the scope of the present invention is 5-chloro-3-(2-thienoyl)oxindole-1-carboxamide, which is in the form of the acid or as anhydrous polymorphous form A of the sodium salt. This compound has the formula III. 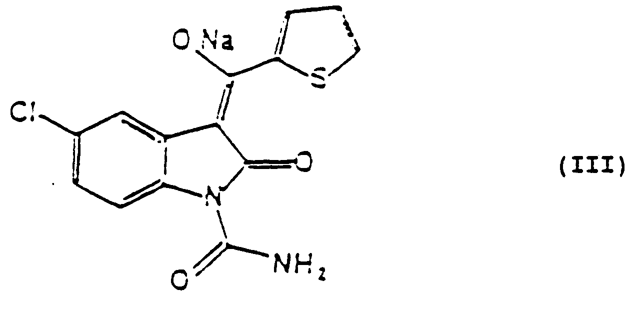
Polyvinylpyrrolidone is used as absorption promoter within the scope of the present invention. In a preferred manner, polyvinylpyrrolidone is employed in an amount of 100-400 per cent by weight and particularly preferably in an amount of 150-300 per cent by weight, based on the weight of the 2-oxindole-1-carboxamide derivative employed.

Polyethylene glycols with an average molecular weight between 400 and 6,000 are also employed as absorption promoters within the scope of the present invention. In this connection, mixtures of various polyethylene glycols with different average molecular weights are particularly preferably employed. The pharmaceutical compositions preferably have 80-99.99 per cent by weight and particularly preferably 86-99 per cent by weight of polyethylene glycol based on the 2-oxindole-1-carboxamide derivative employed.

When polyethylene glycols were used there was found to be a slight irritant effect of the suppository on the rectal mucosa in a few cases. However, it was possible considerably to reduce the irritant effect by adding polyoxyethylene stearate (for example polyoxyl 30 stearate) with a melting point between about 33 and 40°C.

Thus, in another preferred embodiment of the present invention, the pharmaceutical compositions have 10-89.99 per cent by weight of polyethylene glycol, 10-89.99 per cent by weight of polyoxyl 30 stearate and at least 0.01% by weight of 2-oxindole-1-carboxamide derivative.

In a very particularly preferred embodiment, the pharmaceutical compositions have 37-62.99 per cent by weight of polyethylene glycol, 37-62.99 per cent by weight of polyoxyl 30 stearate and at least 0.01% by weight of 2-oxindole-1-carboxamide derivative.

It is also possible to employ a combination of polyethylene glycol and polyvinylpyrrolidone as absorption promoter.

In a preferred embodiment, the pharmaceutical compositions according to the invention are in the form of suppositories, where a single suppository contains 5-180 mg and preferably 20-140 mg of the 2-oxindole-1-carboxamide derivative.

The pharmaceutical compositions according to the invention are prepared by processes known per se, with the agent normally being stirred into a melt. This melt can consist of polyethylene glycol, or of a mixture of polyethylene glycol and polyoxyl 30 stearate.

In the preparation of suppositories with polyvinylpyrrolidone, in a preferred manner the agent and the polyvinylpyrrolidone are suspended in a fatty base as suppository base. Suppositories of this type are particularly well tolerated.

When the absorption promoters according to the invention are used, the rectal bioavailability is increased to values of at least about 90% compared with values on oral administration. Thus, when the absorption promoters according to the invention are employed, it is no longer necessary to incorporate into the suppositories agent concentrations which are considerably higher than the agent concentration in a comparable presentation which can be administered orally.

Furthermore, the irritant effect of the pharmaceutical compositions according to the invention is very slight so that the suppositories according to the invention are very well tolerated.

### Example 1 (preparation example)

128.2 g of 5-chloro-3-(2-thienoyl)oxindole-1-carboxamide sodium salt were added to a stirred melt consisting of 673 g of polyethylene glycol 4000, 224.4 g of polyethylene glycol 1500 and 222.4 g of polyethylene glycol 600. The resulting composition was poured, while stirring, into suppository moulds. It was possible for the suppositories to be used after the melt had cooled.

### Example 2 (preparation example)

128.2 g of 5-chloro-3-(2-thienoyl)oxindole-1-carboxamide sodium salt were added to a stirred melt consisting of 1060.9 g of polyethylene glycol 1500 and 1060.9 g of polyoxyl 30 stearate. The resulting composition was poured, while stirring, into suppository moulds. It was possible to remove the finished suppositories after the melt had cooled.

### Example 3 (preparation example)

128.2 g of 5-chloro-3-(2-thienoyl)oxindole-1-carboxamide sodium salt were suspended together with 300 g of polyvinylpyrrolidone in 1570.4 g of fatty base (Suppocire) in molten form at about 45°C. The suspension was poured, while stirring, into suppository moulds. It was possible for the finished suppositories to be removed after cooling.

### Example 4 (bioavailability comparison)

The pharmacokinetics of the suppositories according to the invention were examined in dogs as a model. Employed for this were, on the one hand, suppositories prepared as in Examples 1-3, a suppository prepared without absorption promoter, and an orally administered solution. This orally administered aqueous solution of the agent represents the standard with which the other investigation results were compared. Suppositories without absorption promoters showed a bioavailability of only about 26% compared with the orally administered solution. This is consistent with the state of the art, from which it is known that about 1.5-3 times an oral dose has to be incorporated into a suppository in order to achieve equally high blood levels as on oral administration.

Table I shows that the suppositories according to the invention have bioavailabilities which are perfectly comparable with oral administration of the agent.

As another parameter, the time required to reach the maximum blood level (Tmax) was measured. This value on oral administration is only about one half of the value achieved by employing a suppository without absorption promoter.

However, when the absorption promoters according to the invention are used, the suppositories prepared as in Examples 1-3 show values which are perfectly comparable with the oral administration form. The value of Tmax also plays an essential part because the agent employed according to the invention also has an analgesic effect, for which reason a long time span until the maximum blood level is reached is not tolerable.

The results of the comparative tests are compiled in Table I which follows:

**Table I**

| Composition | Tmax (hours) | Bioavailability (0-10h) in % |
|---|---|---|
| orally administered solution (standard) | 4.5 | 98 |
| suppository without absorption promoter | 9 | 26 |
| suppository of Example 1 | 6 | 100.5 |
| suppository of Example 2 | 4 | 97.6 |
| suppository of Example 3 | 2.5 | 87 |

## Claims (Claims for the following Contracting State(s): AT, BE, CH, DE, DK, FR, GB, IT, LI, LU, NL, PT, SE)

1. Pharmaceutical composition for rectal administration, characterised in that it contains a 2-oxindole-1-carboxamide derivative and, as absorption promoter, polyethylene glycol or polyvinylpyrrolidone.

2. Pharmaceutical composition according to Claim 1, characterised in that the 2-oxindole-1-carboxamide derivative is a compound of the formula I: 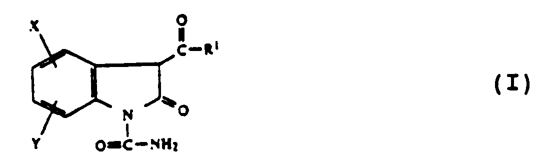 or a pharmaceutically acceptable salt thereof, in which Y is hydrogen or chlorine, X is 5-chloro, 6-chloro, 5-fluoro, 6-fluoro, 5-trifluoromethyl or 6-trifluoromethyl, and in which R¹ is benzyl, 2-furyl, 2-thienyl, (2-furyl)methyl or (2-thienyl)methyl.

3. Pharmaceutical composition according to Claim 2, characterised in that the 2-oxindole-1-carboxamide derivative has the following formula (II) 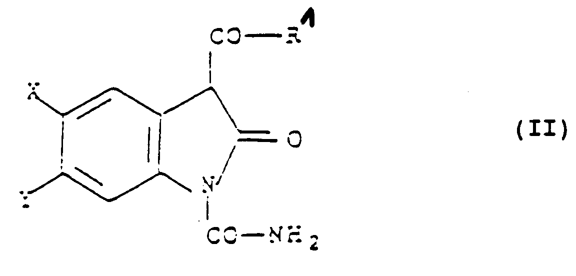 in which the substituents X, Y and R¹ have the following meanings
a) X = Cl, Y = H, R¹ = 2-thienyl or
b) X = F, Y = Cl, R¹ = 2-thienyl or
c) X = H, Y = Cl, R¹ = benzyl.

4. Pharmaceutical composition according to any of the preceding claims, characterised in that 5-chloro-3-(2-thienoyl)oxindole-1-carboxamide in the form of the acid or as anhydrous polymorphous form A of the sodium salt is employed as 2-oxindole-1-carboxamide derivative.

5. Pharmaceutical composition according to any of Claims 1-4, characterised in that it contains 100-400 per cent by weight of polyvinylpyrrolidone, based on the weight of the 2-oxindole-1-carboxamide derivative employed.

6. Pharmaceutical composition according to Claim 5, characterised in that it contains 150-300 per cent by weight of polyvinylpyrrolidone, based on the weight of the 2-oxindole-1-carboxamide derivative employed.

7. Pharmaceutical composition according to any of Claims 1-4, characterised in that it has polyethylene glycol with an average molecular weight of 400 to 6,000.

8. Pharmaceutical composition according to Claim 7, characterised in that it contains 80-99.99 per cent by weight of polyethylene glycol.

9. Pharmaceutical composition according to Claim 7 or 8, characterised in that it contains 86-99.99 per cent by weight of polyethylene glycol.

10. **.** Pharmaceutical composition according to any of Claims 1-4, characterised in that it has 10-89.99 per cent by weight of polyethylene glycol and 10-89.99 per cent by weight of polyoxyl 30 stearate and at least 0.01% by weight of 2-oxindole-1-carboxamide derivative.

11. Pharmaceutical composition according to Claim 10, characterised in that it has 37-62.99 per cent by weight of polyethylene glycol, 37-62.99 per cent by weight of polyoxyl 30 stearate and at least 0.01% by weight of 2-oxindole-1-carboxamide derivative.

12. Pharmaceutical composition according to any of the preceding claims, characterised in that it is in the form of suppositories.

13. Pharmaceutical composition according to Claim 12, characterised in that it contains 5-180 mg of the 2-oxindole-1-carboxamide derivative per suppository.

14. Pharmaceutical composition according to Claim 13, characterised in that it contains 20-140 mg of the 2-oxindole-1-carboxamide derivative per suppository.

## Claims (Claims for the following Contracting State(s): ES, GR)

1. Process for preparing a pharmaceutical composition for rectal administration, characterised in that it comprises admixing a 2-oxindole-1-carboxamide derivative and, as absorption promoter, polyethylene glycol or polyvinyl-pyrrolidone.

2. Process according to Claim 1, characterised in that the 2-oxindole-1-carboxamide derivative is a compound of the formula I: 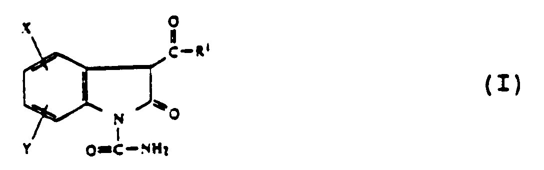 or a pharmaceutically acceptable salt thereof, in which Y is hydrogen or chlorine, X is 5-chloro, 6-chloro, 5-fluoro, 6-fluoro, 5-trifluoromethyl or 6-trifluoromethyl, and in which R¹ is benzyl, 2-furyl, 2-thienyl, (2-furyl)methyl or (2-thienyl)methyl.

3. Process according to Claim 2, characterised in that the 2-oxindole-1-carboxamide derivative has the following formula (II) 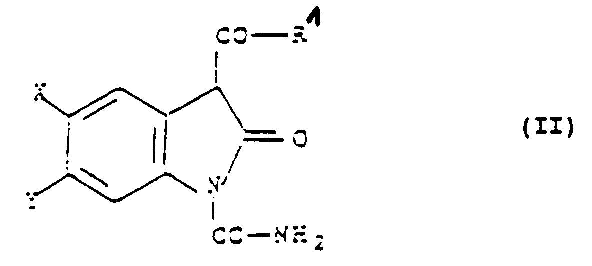 in which the substituents X, Y and R¹ have the following meanings
a) X = Cl, Y = H, R¹ = 2-thienyl or
b) X = F, Y = Cl, R¹ = 2-thienyl or
c) X = H, Y = Cl, R¹ = benzyl.

4. Process according to any of the preceding claims, characterised in that 5-chloro-3-(2-thienoyl)oxindole-1-carboxamide in the form of the acid or as anhydrous polymorphous form A of the sodium salt is employed as 2-oxindole-1-carboxamide derivative.

5. Process according to any of Claims 1-4, characterised in that the pharmaceutical composition contains 100-400 per cent by weight of polyvinylpyrrolidone, based on the weight of the 2-oxindole-1-carboxamide derivative employed.

6. Process according to Claim 5, characterised in that the pharmaceutical composition contains 150-300 per cent by weight of polyvinylpyrrolidone, based on the weight of the 2-oxindole-1-carboxamide derivative employed.

7. Process according to any of Claims 1-4, characterised in that the pharmaceutical composition has polyethylene glycol with an average molecular weight of 400 to 6,000.

8. Process according to Claim 7, characterised in that the pharmaceutical composition-contains 80-99.99 per cent by weight of polyethylene glycol.

9. Process according to Claim 7 or 8, characterised in that the pharmaceutical composition contains 86-99.99 per cent by weight of polyethylene glycol.

10. Process according to any of Claims 1-4, characterised in that the pharmaceutical composition has 10-89.99 per cent by weight of polyethylene glycol and 10-89.99 per cent by weight of polyoxyl 30 stearate and at least 0.01% by weight of 2-oxindole-1-carboxamide derivative.

11. Process according to Claim 10, characterised in that the pharmaceutical composition has 37-62.99 per cent by weight of polyethylene glycol, 37-62.99 per cent by weight of polyoxyl 30 stearate and at least 0.01% by weight of 2-oxindole-1-carboxamide derivative.

12. Process according to any of the preceding claims, characterised in that the pharmaceutical composition is in the form of suppositories.

13. Process according to Claim 12, characterised in that the pharmaceutical composition contains 5-180 mg of the 2-oxindole-1-carboxamide derivative per suppository.

14. Process according to Claim 13, characterised in that the pharmaceutical composition contains 20-140 mg of the 2-oxindole-1-carboxamide derivative per suppository.

## Patentansprüche (Patentansprüche für folgende(n) Vertragsstaat(en): AT, BE, CH, DE, DK, FR, GB, IT, LI, LU, NL, PT, SE)

1. Pharmazeutische Zubereitung zur rektalen Applikation, dadurch gekennzeichnet, daß sie ein 2-Oxindol-1-carboxamid-derivat und als Absorptionsbeschleuniger Polyethylenglykol oder Polyvinylpyrrolidon enthält.

2. Pharmazeutische Zubereitung nach Anspruch 1, dadurch gekennzeichnet, daß das 2-Oxindol-1-carboxamid-derivat eine Verbindung der Formel I: 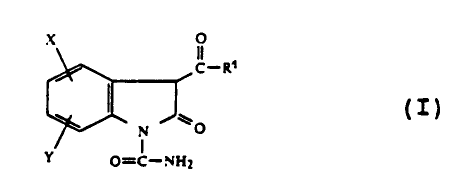 oder ein pharmazeutisch annehmbares Salz davon ist, worin Y Wasserstoff oder Chlor ist, X 5-Chlor, 6-Chlor, 5-Fluor, 6-Fluor, 5-Trifluormethyl oder 6-Trifluormethyl ist und worin R¹ Benzyl, 2-Furyl, 2-Thienyl, (2-Furyl)-methyl oder (2-Thienyl)-methyl ist.

3. Pharmazeutische Zubereitung nach Anspruch 2, dadurch gekennzeichnet, daß das 2-Oxindol-1-carboxamid-derivat die folgende Formel (II) aufweist 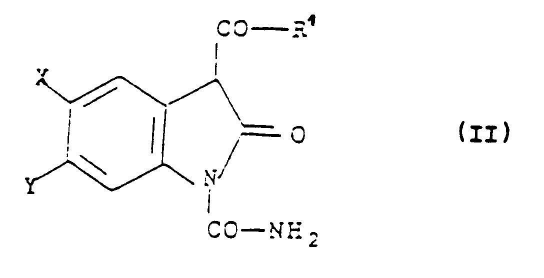 worin die Substituenten X, Y und R¹ folgende Bedeutung haben
a) X = Cl, Y = H, R¹ = 2-Thienyl, oder
b) X = F, Y = Cl, R¹ = 2-Thienyl, oder
c) X = H, Y = Cl, R¹ = Benzyl.

4. Pharmazeutische Zubereitung nach einem der vorhergehenden Ansprüche, dadurch gekennzeichnet, daß als 2-Oxindol-1-carboxamid-derivat das 5-Chlor-3-(2-thienoyl) -oxindol-1-carboxamid in Form der Säure oder als wasserfreie polymorphe Form A des Natriumsalzes eingesetzt wird.

5. Pharmazeutische Zubereitung nach einem der Ansprüche 1 - 4, dadurch gekennzeichnet, daß sie 100 - 400 Gewichtsprozent Polyvinylpyrrolidon enthält, bezogen auf das Gewicht des eingesetzten 2-Oxindol-1-carboxamid-derivats.

6. Pharmazeutische Zubereitung nach Anspruch 5, dadurch gekennzeichnet, daß sie 150 - 300 Gewichtsprozent Polyvinylpyrrolidon enthält, bezogen auf das Gewicht des eingesetzten 2-Oxindol-1-carboxamid-derivats.

7. Pharmazeutische Zubereitung nach einem der Ansprüche 1 - 4, dadurch gekennzeichnet, daß sie Polyethylenglykol mit einem mittleren Molekulargewicht von 400 bis 6000 aufweist.

8. Pharmazeutische Zubereitung nach Anspruch 7, dadurch gekennzeichnet, daß sie 80 - 99,99 Gewichtsprozent Polyethylenglykol enthält.

9. Pharmazeutische Zubereitung nach Anspruch 7 oder 8 , dadurch gekennnzeichnet, daß sie 86 - 99,99 Gewichtsprozent Polyethylenglykol enthält.

10. Pharmazeutische Zubereitung nach einem der Ansprüche 1 - 4, dadurch gekennzeichnet, daß sie 10 - 89,99 Gewichtsprozent Polyethylenglykol und 10 - 89,99 Gewichtsprozent Polyoxyl-30-stearat und wenigstens 0,01 Gew. - % 2-Oxindol-1-carboxamid - derivat aufweist.

11. Pharmazeutische Zubereitung nach Anspruch 10 , dadurch gekennzeichnet, daß sie 37 - 62,99 Gewichtsprozent Polyethylenglykol, 37 - 62,99 Gewichtsprozent Polyoxyl-30-stearat und wenigstens 0,01 Gew. % 2-Oxindol-1-carboxamid-derivat aufweist.

12. Pharmazeutische Zubereitung nach einem der vorhergehenden Ansprüche, dadurch gekennzeichnet, daß sie in Form von Suppositorien vorliegt.

13. Pharmazeutische Zubereitung nach Anspruch 12, dadurch gekennzeichnet, daß sie 5 - 180 mg des 2-Oxindol-1-carboxamid-derivats pro Suppositorium enthält.

14. Pharmazeutische Zubereitung nach Anspruch 13, dadurch gekennzeichnet, daß sie 20 - 140 mg des 2-Oxindol-1-carboxamid-derivats pro Suppositorium enthält.

## Patentansprüche (Patentansprüche für folgende(n) Vertragsstaat(en): ES, GR)

1. Verfahren zur Herstellung einer pharmazeutischen Zubereitung zur rektalen Applikation, dadurch gekennzeichnet, daß es das Mischen eines 2-Oxindol-1-carboxamid-derivats und, als Absorptionsbeschleuniger, Polyethylenglycol oder Polyvinylpyrrolidon umfaßt.

2. Verfahren nach Anspruch 1, dadurch gekennzeichnet, daß das 2-Oxindol-1-carboxamid-derivat eine Verbindung der Formel I: 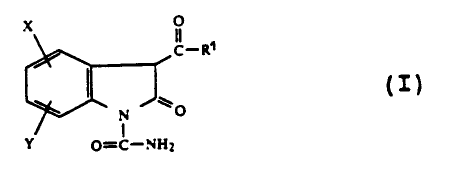 oder ein pharmazeutisch annehmbares salz davon ist, worin Y Wasserstoff oder Chlor ist, X 5-Chlor, 6-Chlor, 5-Fluor, 6-Fluor, 5-Trifluormethyl oder 6-Trifluormethyl ist und worin R¹ Benzyl, 2-Furyl, 2-Thienyl, (2-Furyl)-methyl oder (2-Thienyl)-methyl ist.

3. Verfahren nach Anspruch 2, dadurch gekennzeichnet, daß das 2-Oxindol-1-carboxamid-derivat die folgende Formel (II) aufweist 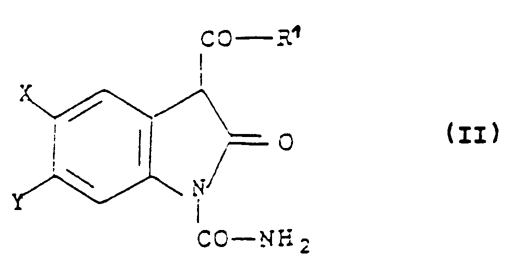 worin die Substituenten X, Y und R¹ folgende Bedeutungen haben
a) X = Cl, Y = H, R¹ = 2-Thienyl, oder
b) X = F, Y = Cl, R¹ = 2-Thienyl, oder
c) X = H, Y = Cl, R¹ = Benzyl.

4. Verfahren nach einem der vorhergehenden Ansprüche, dadurch gekennzeichnet, daß als 2-Oxindol-1-carboxamid-derivat das 5-Chlor-3-(2-thienoyl)-oxindol-1-carboxamid in Form der Säure oder als wasserfreie polymorphe Form A des Natriumsalzes eingesetzt wird.

5. Verfahren nach einem der Ansprüche 1-4, dadurch gekennzeichnet, daß die pharmazeutische Zubereitung 100-400 Gewichtsprozent Polyvinylpyrrolidon enthält, bezogen auf das Gewicht des eingesetzten 2-Oxindol-1-carboxamid-derivats.

6. Verfahren nach Anspruch 5, dadurch gekennzeichnet, daß die pharmazeutische Zubereitung 150-300 Gewichtsprozent Polyvinylpyrrolidon enthält, bezogen auf das Gewicht des eingesetzten 2-Oxindol-1-carboxamid-derivats.

7. Verfahren nach einem der Ansprüche 1-4, dadurch gekennzeichnet, daß die pharmazeutische Zubereitung Polyethylenglycol mit einem mittleren Molekulargewicht von 400 bis 6000 aufweist.

8. Verfahren nach Anspruch 7, dadurch gekennzeichnet, daß die pharmazeutische Zubereitung 80-99,99 Gewichtsprozent Polyethylenglycol enthält.

9. Verfahren nach Anspruch 7 oder 8, dadurch gekennzeichnet, daß die pharmazeutische Zubereitung 86-99,99 Gewichtsprozent Polyethylenglycol enthält.

10. Verfahren nach einem der Ansprüche 1-4, dadurch gekennzeichnet, daß die pharmazeutische Zubereitung 10-89,99 Gewichtsprozent Polyethylenglycol und 10-89,99 Gewichtsprozent Polyoxyl-30-stearat und wenigstens 0,01 Gew.-% 2-Oxindol-1-carboxamid-derivat aufweist.

11. Verfahren nach Anspruch 10, dadurch gekennzeichnet, daß die pharmazeutische Zubereitung 37-62,99 Gewichtsprozent Polyethylenglycol, 37-62,99 Gewichtsprozent Polyoxyl-30-stearat und wenigstens 0,01 Gew.-% 2-Oxindol-1-carboxamid-derivat aufweist.

12. Verfahren nach einem der vorhergehenden Ansprüche, dadurch gekennzeichnet, daß die pharmazeutische Zubereitung in Form von Suppositorien vorliegt.

13. Verfahren nach Anspruch 12, dadurch gekennzeichnet, daß die pharmazeutische Zubereitung 5-180 mg des 2-Oxindol-1-carboxamid-derivats pro Suppositorium enthält.

14. Verfahren nach Anspruch 13, dadurch gekennzeichnet, daß die pharmazeutische Zubereitung 20-140 mg des 2-Oxindol-1-carboxamid-derivats pro Suppositorium enthält.

## Revendications (Revendications pour l'(les) Etat(s) contractant(s) suivant(s): AT, BE, CH, DE, DK, FR, GB, IT, LI, LU, NL, PT, SE)

1. Composition pharmaceutique pour l'administration rectale, caractérisée en ce qu'elle contient un dérivé de 2-oxindole-1-carboxamide et, comme activateur d'absorption, du polyéthylèneglycol ou de la polyvinylpyrrolidone.

2. Composition pharmaceutique suivant la revendication 1, caractérisée en ce que le dérivé de 2-oxindole-1-carboxamide est un composé de formule I : 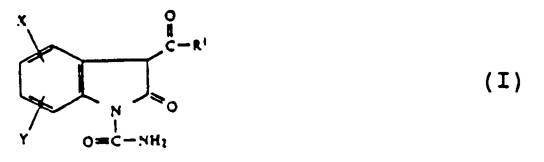 ou un de ses sels pharmaceutiquement acceptables, formule dans laquelle Y représente l'hydrogène ou le chlore, X représente un groupe 5-chloro, 6-chloro, 5-fluoro, 6-fluoro, 5-trifluorométhyle ou 6-trifluorométhyle, et R¹ représente un groupe benzyle, 2-furyle, 2-thiényle, (2-furyl)méthyle ou (2-thiényl)méthyle.

3. Composition pharmaceutique suivant la revendication 2, caractérisée en ce que le dérivé de 2-oxindole-1-carboxamide répond à la formule (II) suivante : 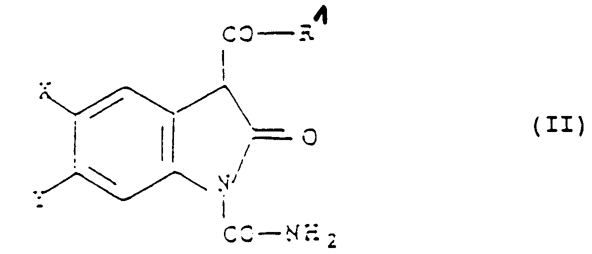 dans laquelle les substituants X, Y et R¹ répondent aux définitions suivantes
a) X = Cl, Y = H R¹ = 2-thiényle ou
b) X = F, Y = Cl, R¹ = 2-thiényle ou
c) X = H, Y = Cl, R¹ = benzyle.

4. Composition pharmaceutique suivant l'une quelconque des revendications précédentes, caractérisée en ce que du 5-chloro-3-(2-thiénoyl)oxindole-1-carboxamide sous forme de l'acide ou de la forme polymorphe anhydre A du sel de sodium est utilisé comme dérivé de 2-oxindole-1-carboxamide.

5. Composition pharmaceutique suivant l'une quelconque des revendications 1 à 4, caractérisée en ce qu'elle contient 100 à 400 % en poids de polyvinyl-pyrrolidone, sur la base du poids du dérivé de 2-oxindole-1-carboxamide utilisé.

6. Composition pharmaceutique suivant la revendication 5, caractérisée en ce qu'elle contient 150 à 300 pour cent en poids de polyvinylpyrrolidone, sur la base du poids du dérivé de 2-oxindole-1-carboxamide utilisé.

7. Composition pharmaceutique suivant l'une quelconque des revendications 1 à 4, caractérisée en ce qu'elle renferme un polyéthylèneglycol ayant un poids moléculaire moyen de 400 à 6000.

8. Composition pharmaceutique suivant la revendication 7, caractérisée en ce qu'elle contient 80 à 99,99 pour cent en poids de polyéthylèneglycol.

9. Composition pharmaceutique suivant la revendication 7 ou 8, caractérisée en ce qu'elle contient 86 à 99,99 pour cent en poids de polyéthylèneglycol.

10. Composition pharmaceutique suivant l'une quelconque des revendications 1 à 4, caractérisée en ce qu'elle renferme 10 à 89,99 pour cent en poids de polyéthylèneglycol et 10 à 89,99 pour cent en poids de polyoxyl-30-stéarate et au moins 0,01 pour cent en poids du dérivé de 2-oxindole-1-carboxamide.

11. Composition pharmaceutique suivant la revendication 10, caractérisée en ce qu'elle renferme 37 à 62,99 pour cent en poids de polyéthylèneglycol, 37 à 62,99 pour cent en poids de polyoxyl-30-stéarate et au moins 0,01 pour cent en poids du dérivé de 2-oxindole-1-carboxamide.

12. Composition pharmaceutique suivant l'une quelconque des revendications précédentes, caractérisée en ce qu'elle est sous forme de suppositoires.

13. Composition pharmaceutique suivant la revendication 12, caractérisée en ce qu'elle contient 5 à 180 mg du dérivé de 2-oxindole-1-carboxamide par suppositoire.

14. Composition pharmaceutique suivant la revendication 13, caractérisée en ce qu'elle contient 20 à 140 mg du dérivé de 2-oxindole-1-carboxamide par suppositoire.

## Revendications (Revendications pour l'(les) Etat(s) contractant(s) suivant(s): ES, GR)

1. Procédé de préparation d'une composition pharmaceutique pour l'administration rectale, caractérisé en ce qu'il comprend le mélange d'un dérivé de 2-oxindole-1-carboxamide et, comme activateur d'absorption, de polyéthylèneglycol ou de polyvinylpyrrolidone.

2. Procédé suivant la revendication 1, caractérisé en ce que le dérivé de 2-oxindole-1-carboxamide est un composé de formule I : 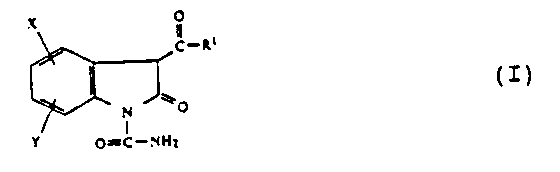 ou un de ses sels pharmaceutiquement acceptables, formule dans laquelle Y représente l'hydrogène ou le chlore, X représente un groupe 5-chloro, 6-chloro, 5-fluoro, 6-fluoro, 5-trifluorométhyle ou 6-trifluorométhyle, et R¹ représente un groupe benzyle, 2-furyle, 2-thiényle, (2-furyl)méthyle ou (2-thiényl)méthyle.

3. Procédé suivant la revendication 2, caractérisé en ce que le dérivé de 2-oxindole-1-carboxamide répond à la formule (II) suivante 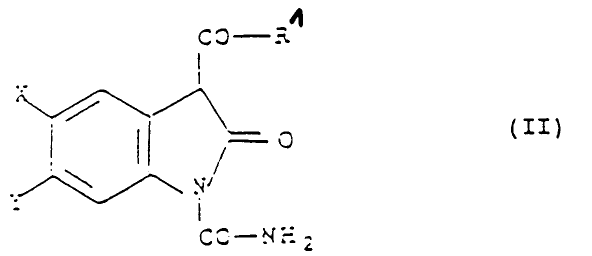 dans laquelle les substituants X, Y et R¹ répondent aux définitions suivantes
a) X = Cl, Y = H, R¹ = 2-thiényle ou
b) X = F, Y = Cl, R¹ = 2-thiényle ou
c) X = H, Y = Cl, R¹ = benzyle.

4. Procédé suivant l'une quelconque des revendications précédentes, caractérisé en ce que du 5-chloro-3-(2-thiénoyl)oxindole-1-carboxamide sous forme de l'acide ou de la forme polymorphe anhydre A du sel de sodium est utilisé comme dérivé de 2-oxindole-1-carboxamide.

5. Procédé suivant l'une quelconque des revendications 1 à 4, caractérisé en ce que la composition pharmaceutique contient 100 à 400 pour cent en poids de polyvinylpyrrolidone, sur la base du poids du dérivé de 2-oxindole-1-carboxamide utilisé.

6. Procédé suivant la revendication 5, caractérisé en ce que la composition pharmaceutique contient 150 à 300 pour cent en poids de polyvinylpyrrolidone, sur la base du poids du dérivé de 2-oxindole-1-carboxamide utilisé.

7. Procédé suivant l'une quelconque des revendications 1 à 4, caractérisé en ce que la composition pharmaceutique renferme un polyéthylèneglycol ayant un poids moléculaire moyen de 400 à 6000.

8. Procédé suivant la revendication 7, caractérisé en ce que la composition pharmaceutique contient 80 à 99,99 pour cent en poids de polyéthylèneglycol.

9. Procédé suivant la revendication 7 ou 8, caractérisé en ce que la composition pharmaceutique contient 86 à 99,99 pour cent en poids de polyéthylèneglycol.

10. Procédé suivant l'une quelconque des revendications 1 à 4, caractérisé en ce que la composition pharmaceutique renferme 10 à 89,99 pour cent en poids de polyéthylèneglycol et 10 à 89,99 pour cent en poids de polyoxyl-30-stéarate et au moins 0,01 % en poids du dérivé 2-oxindole-1-carboxamide.

11. Procédé suivant la revendication 10, caractérisé en ce que la composition pharmaceutique renferme 37 à 62,99 pour cent en poids de polyéthylèneglycol, 37 à 62,99 pour cent en poids de polyoxyl-30-stéarate et au moins 0,01 % en poids du dérivé de 2-oxindole-1-carboxamide.

12. Procédé suivant l'une quelconque des revendications précédentes, caractérisé en ce que la composition pharmaceutique est sous forme de suppositoires.

13. Procédé suivant la revendication 12, caractérisé en ce que la composition pharmaceutique contient 5 à 180 mg de dérivé de 2-oxindole-1-carboxamide par suppositoire.

14. Procédé suivant la revendication 13, caractérisé en ce que la composition pharmaceutique contient 20 à 140 mg du dérivé de 2-oxindole-1-carboxamide par suppositoire.
